Europäisches Patentamt

European Patent Office

Office européen des brevets

(19)

(11) Veröffentlichungsnummer: **0 268 763 B1**

(12) **EUROPÄISCHE PATENTSCHRIFT**

(45) Veröffentlichungstag der Patentschrift: **05.08.92**

(51) Int. Cl.5: **A61K 7/16**, C01B 33/154

(21) Anmeldenummer: **87113005.0**

(22) Anmeldetag: **05.09.87**

(54) **Zahnpflegemittel.**

(30) Priorität: **21.11.86 DE 3639844**

(43) Veröffentlichungstag der Anmeldung:
**01.06.88 Patentblatt 88/22**

(45) Bekanntmachung des Hinweises auf die
Patenterteilung:
**05.08.92 Patentblatt 92/32**

(84) Benannte Vertragsstaaten:
**AT BE CH DE ES FR GB LI NL SE**

(56) Entgegenhaltungen:
EP-A- 0 062 749
DE-B- 1 467 019
FR-A- 2 158 217
FR-A- 2 173 996
US-A- 4 140 757

(73) Patentinhaber: **Hawe-Neos Dental Dr. H. v. Weissenfluh AG**

**CH-6925 Gentilino(CH)**

(72) Erfinder: **Mühlemann, Hans R., Prof.Dr.
Benstweg 8
CH-8032 Zürich(CH)**
Erfinder: **Müller, Karl-Hans, Dr.
Robert Koch-Str. 17
W-6454 Bruchköbel 1(DE)**
Erfinder: **Neumüller, Matthias
Lindenstr. 18
W-6467 Hasselroth 1(DE)**

(74) Vertreter: **Baggiolini, Raimondo et al
Patent Attorneys Fiammenghi-
Fiammenghi-Racheli Via San Gottardo 15
CH-6900 Lugano(CH)**

**Beschreibung**

Die Erfindung betrifft ein Zahnpflegemittel zur vorbeugenden Zahnhygiene.

Ein derartiges Zahnpflegemittel besteht in der Regel aus drei verschiedenen nach Härte eingeordneten Pasten (hart, mittel, weich), deren Struktur nicht mit einer üblicherweise allgemeinverwendeten Zahnpasta vergleichbar ist. Sie entspricht eher einer fast krümeligen Polierpaste. Diese krümelige Struktur ist bei Anwendung in der Mundhöhle mit einem Poliergerät notwendig, um Substanzverlust durch z.B. Spritzen zu vermeiden. Diese Pasten werden in Ergänzung zur täglichen Zahnpflege von Zahnhygienikern benutzt, um hartnäckig anhaftende Zahnbeläge zu entfernen. Diese zusätzliche Behandlung soll die Zahnpflege derart unterstützen, dass Karies verringert und somit zahnärztliche Behandlung weitgehend nahezu überflüssig wird. Erfahrungen haben gezeigt, dass für eine gute Wirkung ein 2-monatiger Reinigungsturnus notwendig ist. Die Mindestanzahl dieser Pflege beträgt 2x im Jahr. Bei der Durchführung dieser zusätzlichen Pflege wird von dem Zahnhygieniker der Zahnbelag zuerst mit der harten, dann mit der mittelharten und anschliessend mit der weichen Paste behandelt. Dabei wird ein Aufrauhen der Zahnoberfläche mit der harten Paste angestrebt, um die Fluorkomponente des Zahnpflegemittel zum Erzielen einer Langzeitwirkung in dieser rauhen Oberfläche einzubetten. Die mittelharte und die Weiche Paste sollen die gröbsten Unebenheit auf dem Zahn wegpolieren.

Bekannte Zahnpasten zur vorbeugeden Zahnhygiene-Prophylaxe enthalten neben Silikaten auch Bimsstein, Zirkonsilikat bzw. hartes $Al_2O_3$.

Damit werden RDA-Werte von 250 (harte Paste), 120 (mittelharte Paste) und 40 (weiche Paste) erreicht. Versuche haben ergeben, dass der Kupferabrieb dieser 3 Varianten mit 70-100 mgCu relativ hoch, aber doch nicht sehr unterschiedlich ist.

Gegenstand der Erfindung ist ein Zahnpflegemittel zur vorbeugenden Zahnhygiene dadurch gekennzeichnet, dass es als Putzkörper eine Mischung, bestehend aus einem Perlit und einer synthetisch hergestellten Fällungskieselsäure enthält, wobei man die Fällungskieselsäure dadurch erhält, dass man die in üblicher Weise auf dem Fällungswege erhaltenen Fällungskieselsäuren unterschiedlicher Teilchengrösse und Teilchendichte in der Suspensionsphase homogen miteinander vermischt und die Gemische in üblicher Weise durch Filtrieren, Waschen, Trocknen und Vermahlen aufarbeitet.

Das erfindungsgemässe Zahnpflegemittel kann neben dem Putzkörper, der 30-50 Gew.-% des gesamten Zahnpflegemittels ausmachen kann, 50-70 Gew.-% eines Lösungsmittelgemisches, bestehend aus Glycerin (86 %) und Wasser im Verhältnis von 1:1, enthalten.

Der Anteil an Fällungskieselsäure kann in dem Putzkörper den Anteil an Perlit übersteigen. Diese Massnahme kann die Einstellung einer mittleren Abrasivität bewirken.

Das Lösungsmittel kann aus Glycerin (86 %) und Wasser im beliebigen Mischungsverhältnis, vorzugsweise 1:1, bestehen.

Der Putzkörper kann überwiegend amorphe Fällungskieselsäure enthalten, um die Abrasivität auf einen niedrigen Wert einzustellen.

Das Verhältnis von Perlit zu Fällungskieselsäure kann je nach gewünschter Abrasivität 1:1 bis 1:6 betragen.

In einer bevorzugten Ausführungsform der Erfindung kann das Zahnpfegemittel eine Fällungskieselsäure mit den folgenden physikalisch-chemischen Kenndaten enthalten:

| Trocknungsverlust (DIN 53 198) | % | 3 - 7 |
|---|---|---|
| Leitfähigkeit bei 25°C ( 4 %ige Aufschlämmung) | µS | 400 - 800 |
| pH-Wert ( 5 % nach DIN 53 200) | | 6 - 7 |
| BET-Oberfläche (DIN 66 131) | m²/g | 130 - 150 |
| Stampfdichte (DIM 53 194) | g/l | 100 - 150 |
| Makroporenvolumen D >30 nm (nach der Hg-Einpreßmethode) | ml/g | 3,2 - 4,0 |
| ölaufnahme n. Gardner | ml/100 g | 180 - 200 |
| Wasserrückhaltevermögen | % | 76 - 79 |
| Cu-Abrieb | mg | 5 - 14 |
| RDA-Abrieb | | 35 - 100 |
| REA-Abrieb | | 40 - 90 |
| Kratzer | | wenig - sehr wenige |
| Viskosität (in 16 %iger Glyzerin-Wasser-Dispersion 1 : 1 Spindel D mit Helipath) | mPas | 5000 - 10 000 |
| Fe-Gehalt | ppm | 240 - 280 |

Insbesondere kann das erfindungsgemässe Zahnpflegemittel eine Fällungskieselsäure mit den physikalisch-chemischen Kenndated

| Trocknungsverlust (DIN 53 198) | % | 3 - 7 |
|---|---|---|
| Leitfähigkeit bei 25°C ( 4 %ige Aufschlämmung) | µS | 400 - 800 |
| pH-Wert ( 5 % nach DIN 53 200) | | 6 - 7 |
| BET-Oberfläche (DIN 66 131) | m²/g | 130 - 150 |
| Stampfdichte (DIN 53 194) | g/l | 100 - 150 |
| Makroporenvolumen D >30 nm (nach der Hg-Einpreßmethode) | ml/g | 3,2 - 4,0 |
| ölaufnahme n. Gardner | ml/100g | 180 - 200 |
| Wasserrückhaltevermögen | % | 76 - 79 |
| Cu-Abrieb | mg | 5 - 14 |
| RDA-Abrieb | | 35 - 100 |
| REA-Abrieb | | 40 - 90 |
| Kratzer | | wenig - sehr wenige |
| Viskosität (in 16 %iger Glyzerin-Wasser-Dispersion 1:1 Spindel D mit Helipath) | mPas | 5000 - 10 000 |
| Fe-Gehalt | ppm | 240 - 280 |

enthalten, welche dadurch hergestellt wird, dass man zum einen eine Verdickungsfällungskieselsäuresuspension durch Umsetzung von Alkalisilikatlösungen mit einer Säure unter Vermeidung einer Gelbindung herstellt, wobei zur Fällung der Kieselsäure in eine vorgelegte Alkalisilikatlösung mit einer Konzentration von etwa 5 bis 25 g $SiO_2$ je Liter Lösung, die Säure und die Alkalisilikatlösung mit bestimmten Lösungskonzentrationen und bestimmten Zulaufgeschwindigkeiten unter Aufrecht erhaltung einer Fälltemperatur im Reaktionsmedium zwischen 80 und 90° C derartig eingespeist werden,dass die Viskosität des Reaktionsmediums in einem Zeitraum von mindestens 30 % der gesamten Fälldauer gleichmässig niedrig und der pH-Wert zwischen 10 und 12 gehalten wird, und die Zugabe der Reaktionsteilnehmer beendet wird, bevor die Viskosität nach Durchlaufen eines Maximums auf einen weniger als 100 % über der Anfangsviskosität liegenden Wert gesunken ist und der pH-Wert mit Schwefelsäure unter 7 eingestellt wird, zum anderen eine Abrasivfällungskieselsäuresuspension herstellt, indem man eine Originalfällungskieselsäuresuspension, die durch Fällung der Kieselsäure aus einer vorgelegten Alkalisilikatlösung mit einer Konzentration von etwa 5 - 25 g $SiO_2$ je Liter Lösung mit einer Säure und Alkalimetallsilikatlösung mit bestimmten Lösungskonzentrationen und bestimmten Zulaufgeschwindigkeiten unter Aufrechterhaltung einer Fälltemperatur im Reaktionsmedium zwischen 80 und 90° C, wobei gegebenenfalls der Behälterinhalt während der gesamten Fälldauer intensiv geschert wird, derart hergestellt wird, dass die Viskosität des Reaktionsmediums in einem Zeitraum von mindestens 30 % der gesamten Fälldauer gleichmässig niedrig und der pH-Wert zwischen 10 und 12 gehalten wird, und die Zugabe der Reaktionsteilnehmer beendet ist, bevor die Viskosität nach Durchlaufen eines Maximums auf einen weniger als 100 % über der Anfangsviskosität

EP 0 268 763 B1

liegenden Wert gesunken ist, den pH-Wert mit Schwefelsäure auf 7 bis 9 einstellt, mit heissem Wasser auf einen Fällungskieselsäuregehalt von 10 - 30 g/l und Natriumsulfatgehalt von 6 - 20g $Na_2SO_4$/l verdünnt, auf 80 - 90° C erwärmt, und unter Konstanthalten dieses pH-Wertes durch gleichzeitigen Zulauf von Alkalimetallsilikatlösung, Schwefelsäure und gegebenenfalls heissen Wasser über eine Fälldauer von 15 bis 180 Minuten eine Fällungskieselsäureendkonzentration von 40 bis 80 g/l einstellt, die Suspension mit Schwefelsäure auf einen pH-Wert unter 7 ansäuert, beide Fällungskieselsäuresuspensionen miteinander vermischt, die Fällungskiesalsäure abfiltriert, wäscht, spühtrocknet und anschliessend mit der Luftstrahlmühle vermahlt.

In einer bevorzugten Ausführungsform der Erfindung wird die Verdickungskieselsäuresuspension im wesentlichen gemäss DE-AS 14 67 019 hergestellt.

Abrasivfällungskieselsäuresuspension wird vorzugsweise im wesentlichen gemäss DE-OS 31 14 493 Beispiel 10 hergestellt.

Es wurde nun gefunden, dass man durch Mischen von Fällungskieselsäuren unterschiedlicher Eigenschaften in der Suspensionsphase zu besonders wertvollen Fällungskieselsäuren für den Einsatz in Zahnpflegemitteln kommt. Geeignete Fällungskieselsäuresuspensionen sind direkt durch Fällung oder durch Wideraufschlämmung von Fällungskieselsäurefilterpressenteig erhältlich. Vorzugsweise werden direkt durch Fällung hergestellte Fällungskieselsäuresuspensionen verwendet.

Die durch das erfindungsgemässe Mischen von Fällungskieselsäuresuspensionen durch Filtrieren, Trocknen und Vermahlen gewonnenen Fällungskieselsäuren haben in Zahnpflegemitteln günstigere Eigenschaften als Trockenmischung derselben Ausgangsfällungskieselsäure im gleichen Verhältnis.

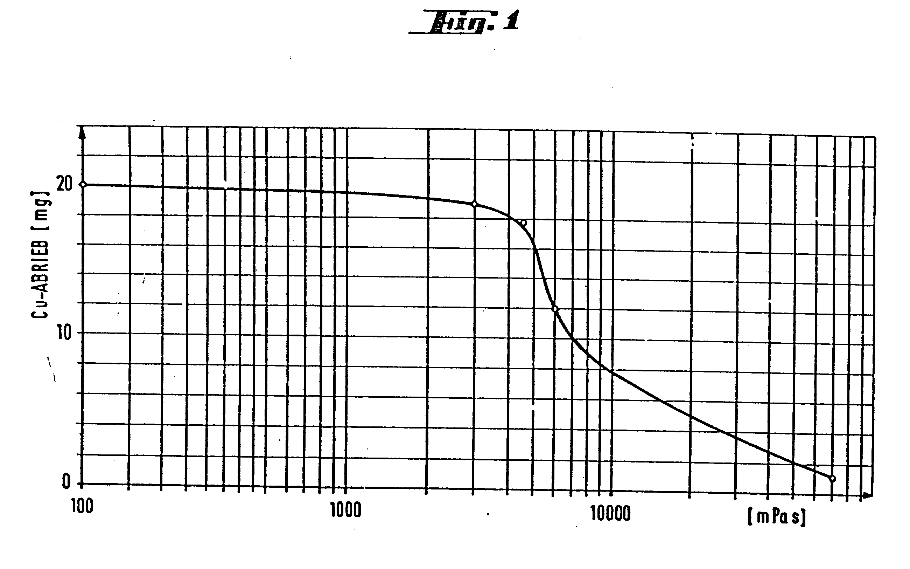

Die erfindungsgemäss eingesetzen Fällungskieselsäuren zeigen synergistische Effekte derart, dass bei gegebener Viskositäts-bzw. Verdickungswirkung eine unerwartet hohe Abrasivität auftritt, d.h., die Viskositäts-Abrasivitätsbeziehung verläuft überraschenderweise nicht linear (vgl. Figur 1).

Die erfindungsgemäss eingesetzten Fällungskieselsäuren lassen sich vorteilhafterweise besonders leicht herstelllen, weil die Mengen über geeignete Volumen-oder Gewichtsmessungen bei bekannten Feststoffgehalten kontinuierlich oder chargenweise exakt eingestellt werden können. Ferner wirft der Nassprozess vorteilhafterweise keine Staubprobleme auf. Auch Entmischungserscheinungen sind minimiert.

Anscheinend verlieren in Nassmischungen im Unterschied zu Trockenmischungen die Komponenten einen Teil ihrer Identität. Beim Vermischen und vermutlich besonders beim Trocknen kommt es zur Wechselwirkungen kleiner mit grösseren Teilchen, wobei die kleinen Teilchen an grössere Teilchen angelagert werden oder aufwachsen.

Auch verschiedene Formen gegenseitiger Flockung und Durchdringung in der wässrigen Phase sind in Betracht zu ziehen.

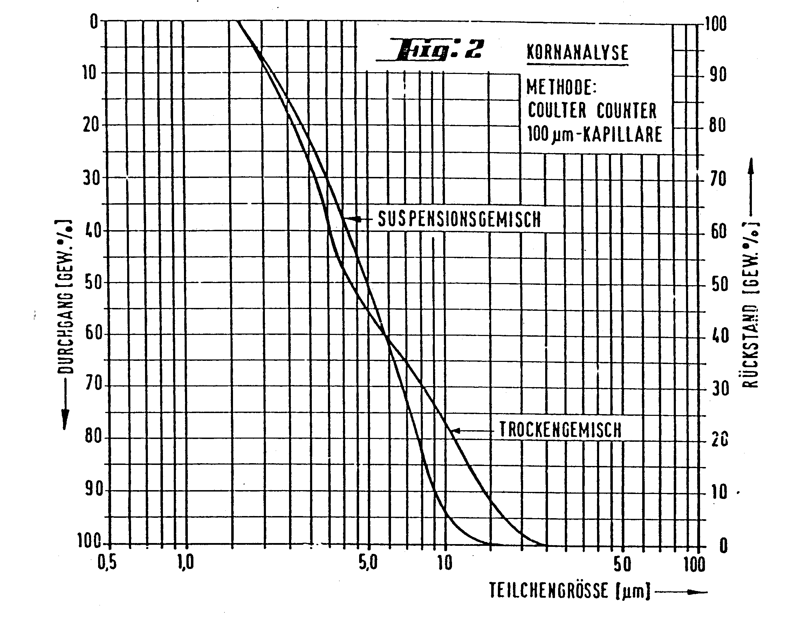

Eine erste deutliche Unterscheidung zwischen Trocken-und Nassmischung ergibt sich aus der Teilchengrössenverteilung der gemahlenen Proben. Während die Trockenmischung zweier Fällungskieselsäuren mit unterschiedlicher Korngrösse und Dichte bei der Vermessung im Coulter Counter erwartungsgemäss 2 Maxima ziegt und die entsprechende Kurve einen Höcker aufweist, verläuft die Kurve der gleichen Fällungskieselsäuren, im gleichen Verhältnis nass miteinander gemischt, vollkommen harmonisch (vgl. Figur 2).

Ein weiterer merklicher Unterschied besteht in der Verdickungswirkung. Die erfindungsgemässe Fällungskieselsäure erhöht die Viskosität einer Modellmischung aus Glyzerin und Wasser deutlich weniger als das Trockengemisch.

Fällungskieselsäuren mit mässiger Verdickungskraft erlauben höhere Füllungsgrade und damit die Herstellung von Pasten mit "vollem" Charakter.

Niedrig gefüllte Pasten wirken demgegenüber in Mund "leer" bzw. "wässrig".

Die verdickende Wirkung der Fällungskieselsäure muss sich bei der Einarbeitung in die Zahnpasta-Masse voll entfalten, d.h. es darf danach keine Veränderung des rheologischen Verhaltens der Zahnpasta auftreten, die das Befüllen und spätere Entleeren der Tuben und Behältnisse erschwert oder gar unmöglich macht.

Grundsätzlich soll ein Zahnpflegemittel die Reinigungswirkung der Zahnbürste unterstützen bzw. erhöhen, indem zwischen Borste und Zahnoberfläche ein Film aufgebaut wird, der Zahnbeläge mechanisch entfernt.

Diese Leistung kann nur von einem Mittel mit abrasiven Eigenschaften erbracht werden.

Bei gleicher REA-Abriebleistung sind selbstverständlich Fällungskieselsäuren zu bevorzugen, die die wenigsten Kratzer erzeugen.

Die erfindungsgemässen Fällungskieselsäuren vereinigen in vorteilhafter Weise Forderungen nach stabiler Viskositätsentwicklung in mittlerer Höhe mit hoher Abrasiv-und Reinigungswirkung unter Vermeidung einer Kratzer.

Die Messung der Abrasivität wir bei extrahierten, radioaktiv gemachten menschlichen Zähnen an

4

freigelegtem Zahnbein (RDA-Wert) oder an Zahnschmelz (REA-Wert) durchgeführt. Gemessen wird jeweils die Zunahme der Radioaktivität der Suspension des zu prüfenden Zahnpflegemittels nach definierter Bearbeitung der Probezähne mit Bürsten.

Ersatzweise kann die Gewichtsabnahme von Kupferplatten beim Bürsten mit Prüfsuspension bestimmt werden. Die so erhaltenen Werte lassen sich aber häufig nicht mit RDA-und REA-Werten vergleichen.

In Speziallaboratorien erhält man mit verfeinerten, z.B. mikroskopischen, Methoden Aufschluss über die reinigende und polierende Wirkung von Zahnpflegemitteln, wobei Proben, die viele oder tiefe Kratzer verursachen, ausgeschieden werden. Diese Untersuchungen werden an extrahierten tierischen oder menschlichen Zähnen durchgeführt.

Beispiele

Der Feststoffgehalt (Fällungskieselsäuregehalt) der Fällungskieselsäuresuspension wird wie folgt bestimmt:

250 ml der auf 20° C abgekühlten Fällungskieselsäuresuspension werden auf einer Porzellannutsche (∅ 120 mm) abgenutscht und danach der Filterkuchen mit 500 ml Wasser elektrolytarm gewaschen. Der Kuchen wird bei 105° C bis zur Gewichtskonstanz getrocknet.

Berechnung: g Auswaage x 4 = g Feststoff/l

Beispiel 1

Es wird eine Fällungskieselsäure mit Verdickungswirkung im wesentlichen gemäss DE-AS 14 67 019 hergestellt, in Abänderung hierzu wird aus ökonomischen Gründen konzentrierte Natriumsilikätlösung (Dichte = 1,353 g/ml) und konzentrierte Schwefelsäure (96 %) eingesetzt. Hieraus resultiert ein Feststoffgehalt von 85 g/l in der Fällungskieselsäuresuspension.

In einem gummierten 120 l-Fällgefäss werden 73 l heissen Wasser und 5,25 l Natriumsilikatlösung (Dichte $\rho$ = 1,353 g/ml, Modul $SiO_2$ : $Na_2O$ = 3,46) unter Rühren auf 85° C erhitzt. In diese alkalische Fällungsvorlage werden während der folgenden 90 Minuten unter Rühren und Aufrechterhaltung der Temperatur von 85° C gleichzeitig 16,5 l Natriumsilikatlösung (Dichte $\rho$ = 1,353 g/ml, Modul $SiO_2$ : $Na_2O$ = 3,46) mit 11,0 l/Stunde und 1,448 l Schwefelsäure (96 % ig) mit 0,965 l/Stunde dosiert. Die Zugabe der Reaktionsteilnehmen wird beendet, bevor die Viskosität des Reaktionsmediums nach Durchlaufen eines Maximums auf einen weniger als 100 % über der Anfangsviskosität liegenden Wert gesunken ist.

Danach wird die so erhaltene Fällungskieselsäuresuspension mit Schwefelsäure (96 % ig) auf einen pH-Wert von 3,5 eingestellt, was durch einen mehrere Minuten lang andauernden Säurezufluss mit 1,25 l/Stunde geschieht. Die auf diese Weise erhaltene Fällungskieselsäuresuspension hat einen Feststoffgehalt von 85,0 g/l.

Die nach dem Abfiltrieren, Auswaschen, Sprühtrocknen und Vermahlen auf der Luftstrahlmühle erhaltene Fällungskieselsäure hat die in der Tabelle 1 aufgeführten physikalisch-chemischen Kenndaten.

Beispiel 2

Das Beispiel beschreibt die Herstellung einer Abrasivfällungskieselsäure gemäss DE-OS 31 14 493, Beispiel 10, jedoch mit der Abänderung, dass die gesamte Wassermenge zur Fällungsvorlage gegeben wird, wodurch die Wasserzugabe während der Fällung entfällt, und die Fällzeit von 60 auf 100 Minuten verlängert wird.

In einem gummierten 120 l-Fällgefäss werden 73 l heisses Wasser und 5,25 l Natriumsilikatlösung (Dichte $\rho$ = 1,353 g/ml; Modul: $SiO_2$ : $Na_2O$ = 3,46) unter Rühren auf 85° C erhitzt. In diese alkalische Fällungsvorlage werden während der folgenden 90 Minuten unter Rühren und Aufrechterhaltung der Temperatur von 85° C gleichzeitig 16,5 l Natriumsilikatlösung (Dichte $\rho$ = 1,353 g/ml; Modul $SiO_2$:$Na_2O$ = 3,46) mit 11,0 l/h und 1,448 l konzentrierte Schwefelsäure (96 % ig) mit 0,965 l/h dosiert. Die Zugabe der Reaktionsteilnehmer wir beendet, bovordie Viskosität des Reaktionsmediums nach Durchlaufen eines Maximums auf einen weniger als 100 % über der Anfangsviskosität liegenden Wert gesunken ist.

Danach wird die so erhaltene Fällungskieselsäuresuspension mit konzentrierter Schwefelsäure (96 % ig) auf einen pH-Wert von 8,5 eingestellt, was durch einen mehrere Minuten lang andauernden Säurezufluss mit 1,25 l/h geschieht. Die auf diese Weise erhaltene Fällungskieselsäuresuspension hat einen Feststoffgehalt von 85 g/l.Ihr $Na_2SO_4$ Gehalt liegt bei 55 g/l.

Während der gesamten Fälldauer von 90 Minuten wird mittels einer Kreiselpumpe, die den Behälterinhalt mehrfach umwälzt, intensiv geschert. Nähere Angaben über die apparative Einrichtung sowie über die Scherbedingungen finden sich in der DE-PS 17 67 332 und dort insbesondere in Spalte 8, Zeilen 31 - 68.

Die auf diese Weise hergestellte Originalfällungskieselsäuresuspension wird mit Wasser auf einen Fällungskieselsäuregehalt von 13 g/l und 8,5 g $Na_2SO_4$/l eingestellt. 16.06 l dieser Suspension werden in einem gummierten 120 l-Fällgefäss unter Rühren auf 85° C erhitzt. Unter Aufrechterhaltung dieser Temperatur und eines pH-Wertes von 8,5 werden für die Dauer von 100 Minuten gleichzeitig 28,1 l Wasserglaslösung (Dichte = 1,353 g/ml; Modul $SiO_2$ : $Na_2O$ = 3,46) mit einer Geschwindigkeit von 231.0 ml/min und 1,94 l Schwefelsäure (96 % ig) mit einer Geschwindigkeit von 19,4 ml/min. zur Fällungskieselsäuresuspension hinzugegeben. Die Fällungskieselsäuresuspension wird anschliessend mit Schwefelsäure (96 % ig) auf einen pH-Wert von ca. 3,5 eingestellt. Die erhaltene Fällungskieselsäuresuspension hat einen Feststoffgehalt von 92,0 g/l.

Die nach dem Abfiltrieren und Auswaschen erhaltene Fällungskieselsäure hat die in der Tabelle 1 aufgeführten physikalisch-chemischen Kenndaten:

Beispiel 3

18,05 m³ Suspension der Abrasivfällungskieselsäuresuspension mit einem Fällungskieselsäuregehalt von 92,0 g/l gemäss Beispiel 2 werden mit 40,0 m³ Suspension einer Verdickungsfällungskieselsäure gemäss Beispiel 1 mit einem Fällungskieselsäuregehalt von 85,0 g/l entsprechend einem Gewichtsverhältnis von 1:2 (bezogen auf den Fällungskieselsäuregehalt) gemischt.

Zur Weiterverarbeitung wird die Fällungskieselsäure dieses Suspensionsgemisches mittels Filterpressen abgetrennt. Der Fällungskieselsäuregehalt im Filterpressenteig beträgt 24 %. Der gewaschene und sprühgetrocknete Filterpressenteig wird luftstrahlvermahlen. Die erhaltene Fällungskieselsäure weist die in der Tabelle 1 aufgeführten physikalisch-chemischen Kenndaten auf.

Tabelle 1

| | | Beisp. 1 | Beisp. 2 | Beisp. 3 |
|---|---|---|---|---|
| Trocknungsverlust (DIN 53 198) | % | 5 | 5 | 5 |
| Leitfähigkeit bei 25° C (4 %ige Aufschlämmung) | μS | 730 | 460 | 740 |
| pH-Wert ( 5 % nach DIN 53 200) | | 6,3 | 6,9 | 6,3 |
| BET-Oberfläche (DIN 66 131) | m²/g | 185 | 40 | 140 |
| Stampfdichte (DIN 53 194) | g/l | 80 | 240 | 130 |
| Makroporenvolumen D>30 nm (nach der Hg-Einpraßmethode) | ml/g | 3,8 | 1,1 | 3,0 |
| Ölaufnahme n. Gardner | ml/100g | 280 | 80 | 184 |
| Wasserrückhaltevermögen | % | 80 | 56 | 76 |
| Cu-Abrieb | mg | 1 | 20 | 12 |
| RDA-Abrieb | | 23 | 170 | 80 |
| REA-Abrieb | | 25 | 130 | 70 |
| Kratzer | | sehr wenige | viel | wenige |
| Viskosität ( in 15 %iger Wasser-Dispersion 1:1 Spindel D mit Helipath) | mPas | 72 000 | < 100 | 6000 |
| Fe-Gehalt | ppm | 260 | 240 | 240 |

Die physikalisch-chemischen Kenndaten, die in der Anmeldung angegeben werden, werden wie folgt bestimmt: Trocknungsverlust nach DIN 53 198.

Leitfähigkeit bei 25° C
4 %ige Aufschlämmung

Eine Probe von 4,0 g wird mit 50 ml vollentsalztem Wasser in einem 150 ml-Becherglas erhitzt und eine Minute unter Rühren gekocht. Anschliessend wird die Suspension in einen 100 ml-Kolben überführt, abgekühlt und mit vollentsalztem Wasser bis zur Marke aufgefüllt. Die elektrische Leitffähigkeit wird mit einem handelsüblichen Messgerät, z.B. der "Wissenschaftlich-Technischen Werkstätten" (WTW), Konduktometer Typ LF 530, bei 25° C gemessen. pH-Wert in 5 % wässriger Dispersion nach DIN 53 200 BET-Oberfläche gemäss DIN 66 131.

Eine Probe wird bei 100° C mit Stickstoff gespült. Die volumetrische Messung wird mit Reinststickstoff bei der Temperatur des flüssigen Stickstoffes (-195,8° C) durchgeführt. Stampfdichte gemäss DIN 53 194.

Die Untersuchung wird ohne Vorbehandlung der Probe vorgenommen.

Makroporenvolumen D ≥ 30 mm nach der Hg-Einpressmethode.

Die Messung erfolgt mit dem Mercury Pressure Porosimeter, 200 Series, der Fa. Carlo Erba Strumentazione.

Oelaufnahme n. Gardner-Coleman
gemäss ASTM : D 1483-60

Wasserrückhaltevermögen

Das Wasserrückhaltevermögen zeigt den, nach Abpressen von Fällungskieselsäuresuspension und Auswaschen auf Elektrolytarmut, enthaltenen Wasseranteil im Pressenteig auf. Zur Bestimmung werden 250 g Pressenteig bis zur Gewichtskonstanz bei 105° C getrocknet.

Berechnung:

$$100 - \frac{Auswaage \times 100}{Einwaage} = \% \ Wasserrückhaltevermögen$$

Teilchengrössenverteilung

gemäss Coulter Conter/100 μm Kap.

Die Messung erfolgt mit dem Coulter Conter-Modell TA 11 der Fa. Coulter Eletronies GmbH.

Bestimmung des Cu-Abreib in 10 % iger Glyzerindispersion

a) Herstellung der Glyzerindispersion

153 g Glyzerin (wasserfrei, reinst pH Eur, BP USP, Dichte = 1,26 g/ml, Fa. Merck, Darmstadt), werden in einem Polyäthylenbecher (250 ml) eingewogen. Mit dem Spatel werden vorsichtig 17 g Fällungskieselsäure untergemischt. Die Mischung wird anschliessend mit einem Flügelrührer (Durchmesser 5,2 cm) 12 Minuten lang bei 1.500 Upm homogenisiert.

b) Durchführung der Abriebmessung

Die Bestimmung der Abriebmessung erfolgt in dem Abriebtestgerät, welches gemäss den folgenden Druckschriften bekannt ist: (1) Pfrengle: Fette, Seifen, Anstrichmittel, 63 (5) (1961), Seiten 445 bis 451 "Abrasion und Reinigungskraft von Putzkörpern in Zahnpasten" (2) A. RENG. F. DANY: Parfümerie und Kosmetik 59 (2) (1978), Seiten 37 bis 45: "Anwendungstechnische Prüfung von Zahnpasten". Dazu werden die 6 Tröge des Testgerätes mit je 20 ml der homogenen Dispersion beschichtet. Der Abrieb, den sechs plangeschliffene Nylonbürsten an sechs plangeschliffenen Cu-Blechen (Elektrolyt-Kupfer) in fünf Stunden mit 50.000 Doppelhüben bewirken, wird durch Differenzwägung bestimmt. Bei der Berechnung der Abrasivität werden von den erhaltenen Werten Mittelwerte gebildet. Der Abrieb (Abrasivität) wird in mg Cu angegeben

RDA-Abrieb
REA-Abrieb
Die RDA-Methode wird beschrieben in Journal of Dental Research, 55 (4), 563 (1976), und wird auch für den REA-Abriebtest angewandt. Kratzer werden visuell mittels Mikroskop bestimmt.

Bestimmung der Viskosität gemessen in 16 %iger Glyzerin/Wasser-Dispersion (Gemisch 1:1) mit Broockfield-Viskosimeter RTV Spinde 10 mit Helipath bei 10 UpM.

1. Probeansätze

| 48 g | Kieselsäure |
|------|-------------|
| 126 g | Glyzerin (etwa 87%, reinst Ph Eur. BP, Dichte = 1,23 g/ml Fa. Merck, Darmstadt |
| 126 g | Wasser dest. |
| 300 g | 16 %ige Dispersion bezogen auf Kieselsäure |

2. Durchführung

Die Abrasivkieselsäuren wurden von Hand in einem 400 ml-Becherglas (breite Form) in das Glyzerin/Wasser-Gemisch mit einem Glasstab eingerührt (1 Minute) und 24 Stunden stehen gelassen. Danach wird die Viskosität gemessen.

3. Messung

Die Viskositätsmessung wird in demselben Becherglas mit dem Brookfield-Viskosimeter RVT, Spindel 10, mit Helipath bei 10 UpM durchgeführt.

4. Ausrechnung

Abgelesener Skalenwert x Faktor = Viskosität in mPas.

Pasten zur Prophylaxe (gemäss Erfindung)

1. Zusammenmischung der pulverförmigen Komponenten im Turbulamischer (5 Min.)
2. Herstellung des Lösungsmittelgemisches Glyzerin (86 %) und destilliertes Wasser im Verhältnis 1:1.

3. Herstellung der Pasten

Die pulverförmigen Komponenten werden in einem 600 ml Becherglas vorgelegt, auf einer Waage tariert und so lange das Lösungsmittelgemisch portionsweise mit einem Spatel eingerührt bis eine der Nupro-Pasten ähnliche Konsistenz erreicht ist, anschliessend Zugabe von 1 % Aromaöl (Pefferminz). Zur Homogenisierung wurde dann die Mischung nochmals durch ein 1 mm Sieb passiert. Die Zusammensetzung der Paste errechnet sich dann aus der Einwaage des Trägers und der zugegebenen Lösungsmittelmenge.

Als Fällungskieselsäure wird die Fällungskieselsäure gemäss Beispiel 3 eingesetzt.
Perlit 2000 ist ein Perlit mit den folgenden physikalisch-chemischen Kenndaten:

| Farbe weiss Schüttgewicht | 80,0 g/l |
|---------------------------|----------|
| Wassegehalt | 0,5 % |
| Glühverlust | 1,0 % |
| PH-Wert | 8,5 |
| Wet Density | 150,0 g/l |
| Durchlaufwert | 450,0 ml |
| Grobteile, nicht anschwemmbar | 1,0 % |

| Korngrössenverteilung/Particles-Size | | | | | | | | | | |
|---|---|---|---|---|---|---|---|---|---|---|
| µm | < 2 | 2-6,3 | 6,3-10 | 10-20 | 26-45 | 45-63 | 63-90 | 90-125 | 125-200 | >200 |
| % | 1 | 4 | 5 | 15 | 10 | 25 | 15 | 10 | 10 | 5 |

| Chemische Analyse/Chemical Analysis | |
|---|---|
| $SiO_2$ | 75,0 % |
| $Fe_2O_3$ | 2,0 % |
| $Al_2O_3$ | 13,0 % |
| CaO | 0,5 % |
| $K_2O$ | 4,0 % |
| MgO | Spuren % |
| $Na_2O$ | 5,0 % |

Die hergestellten Pasten sind in der Tabelle 2 zusammengestellt:

Tabelle 2

| Paste | 1 | 2 | 3 | 4 | 5 | 6 | 7 |
|---|---|---|---|---|---|---|---|
| | harte Paste | | | | | weiche Paste | |
| Perlit 2000 | 22.00 | 13.00 | 9,50 | 7,20 | 6,00 | 5,00 | - |
| Fällungskieselsäure | 22,00 | 26,00 | 28,50 | 28,80 | 30,00 | 30,00 | 35,00 |
| Wasser entsalzt | 22,00 | 27,00 | 28,00 | 30,00 | 30,00 | 31,00 | 31,00 |
| Glyzerin | 33,00 | 33,00 | 33,00 | 33,00 | 33,00 | 33,00 | 33,00 |
| Armoaöl | 1,00 | 1,00 | 1,00 | 1,00 | 1,00 | 1,00 | 1,00 |
| | 100,00 | 100,00 | 100,00 | 100,00 | 100,00 | 100,00 | 100,00 |

Verhältnis von Perlit 2000 zu Fällungskieselsäure in den Pasten

| Paste 1 | Perlit 2000 | Fällungskieselsäure = 1:1 |
|---|---|---|
| Paste 2 | Perlit 2000 | Fällungskieselsäure = 1:2 |
| Paste 3 | Perlit 2000 | Fällungskieselsäure = 1:3 |
| Paste 4 | Perlit 2000 | Fällungskieselsäure = 1:4 |
| Paste 5 | Perlit 2000 | Fällungskieselsäure = 1:5 |
| Paste 6 | Perlit 2000 | Fällungskieselsäure = 1:6 |
| Paste 7 | Perlit 2000 | Fällungskieselsäure = 0:1 |

Die Pasten 1 und 2, die ein Verhältnis von Perlit zu Fällungskieselsäure von 1:1 bis 1:2 aufweisen, sind harte Pasten.

Die Pasten 3 und 4, die ein Verhältnis von Perlit zu Fällungskieselsäure von 1:3 bis 1:4 aufweisen, sind mittelharte Pasten.

Die Pasten 5 und 6, die ein Verhältnis von Perlit zu Fällungskieselsäure von 1:5 bit 1:6 aufweisen, sind weiche Pasten.

Die Pasten 7, die kein Perlit enthält, ist zu weich.

Pasten, die dieses Mischungsverhältnis nicht aufweisen, sind nicht lagerstabil.

Beschreibung der Pasten

Perlit 2000 ist die abrasivere Komponente im Vergleich zur Fällungskieselsäure, hat aber nahezu keine Saugfähigkeit bzw. Adsorptionsfähigkeit. So besteht nicht die Möglichkeit, das Verhältnis Perlit 2000 : Fällungskieselsäure 1 : 1 zugunsten Perlit 2000 zu verschieben, da die Paste sonst instabil wird und Flüssigkeit seporiert und das Sediment sehr hart wird. Im umgekehrten Fall kann aber eine Paste mit der Fällungskieselsäure allein hergestellt werden.

Perlit 2000 hat eine hohe Abrasivität und verursacht deshalb Riefen auf der Zahnoberfläche. Mit der Fällungskieselsäure als Putzkörper mittlerer Abrasivität kann der Riefenwert entscheidend verbessert werden.

Auswertungsdiagramme von Kratzwertbestimmungen handelsüblicher Prophylaxepasten (Nupro) und Fällungskieselsäure/Perlit 2000 Pasten

Die Nupro-Pasten wirken auf den Zahnschmelz sehr viel ungünstiger. Die Auswertung des Riefen-bzw. Kratzwertes der einzelnen Pasten ist wie folgt:

| Pasten | Kratzwert n.30 min. | RDA-Wert | Kupferabrieb (mgCu) |
|---|---|---|---|
| Nupro-hart | 800 | nicht bekannt | 95 |
| Nupro-mittelhart | 367 | nicht bekannt | 85 |
| Nupro-weich | 212 | nicht bekannt | 75 |
| Fällungskieselsäure/ P 2000 hart |  | 348 | 260 |
| Fällungskieselsäure/ P 2000 mittelhart | 122 | 266 | 380 |
| Fällungskieselsäure/ P 2000 weich | 110 | 188 | 450 |
|  |  |  | 26 |

### Kratzwert

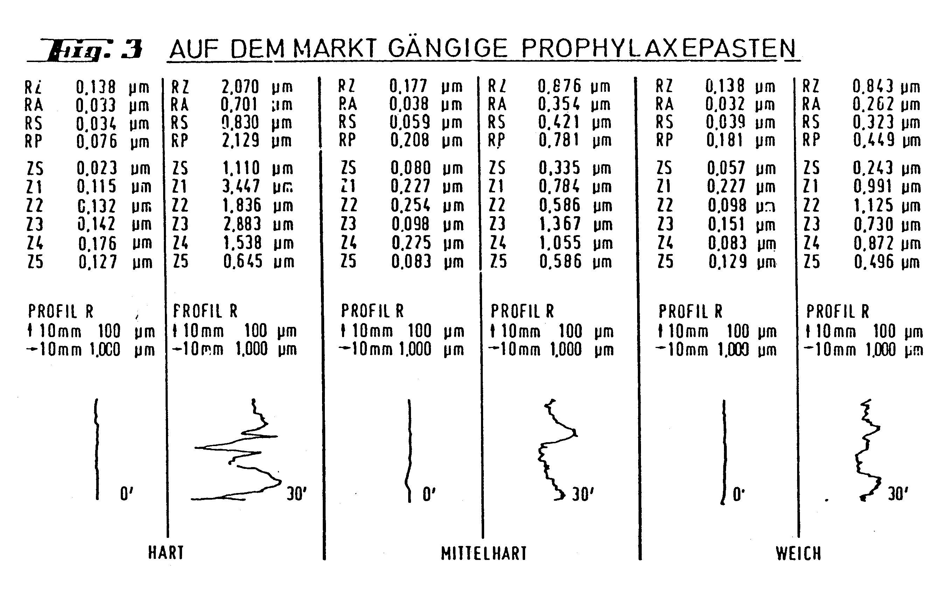

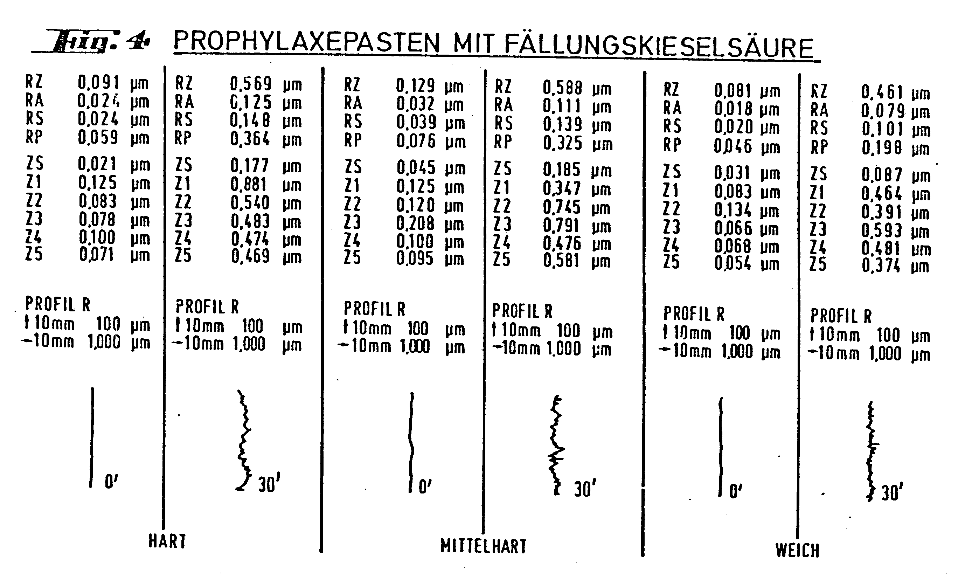

Ein Kratzwert von 100 ± 20 wird für Prophylaxepasten toleriert. Im Vergleich dazu haben die erfindungsgemässen Fällungskieselsäurepasten Werte um 20 - 60. Der Riefenwert wird in der Literatur als Mittenrauhigkeitswert (RA) ermittelt. Die Messungen werden an jeweils 4 Zähnen durchgeführt, wobei ein definierter Vergleich in die Versuche einbezogen wird. Bereits anhand der Kurven (Figur 3 und Figur 4) ist zu sehen, dass sich die Nupro-Pasten wesentlich ungünstiger im Vergleich zu den erfindungsgemässen Pasten verhalten.

### Kupferabrieb

Betrachtet man den Kupferabrieb, bleibt festzustellen, dass die Reinigungskraft der erfindungsgemässen Pasten deutlich höher gegenüber den Nupro-Pasten ist, und dennoch zeigt die Kratzbestimmung, dass eine schonendere Behandlung zu erwarten ist.

### RDA-Wert

Aus diesen Ergebnissen kann abgeleitet werden, dass die mit erfindungsgemässen Prophylaxepasten den Vorteil gegenüber bereits bekannten und marktgängigen Produkten haben, dass sie deutlich höhere Reinigungskraft aufweisen und dabei noch schonender auf den Zahnschmelz einwirken.

**Patentansprüche**

1. Zahnpflegemittel zur vorbeugenden Zahnhygiene dadurch gekennzeichnet, dass es als Putzkörper eine Mischung, bestehend aus einem Perlit und einer synthetisch hergestellten Fällungskieselsäure enthält, wobei man die Fällungskieselsäure dadurch erhält, dass man die in üblicher Weise auf dem Fällungswege erhaltenen Fällungskieselsäuren unterschiedlicher Teilchengrösse und Teilchendichte in der Suspensionsphase homogen miteinander vermischt und die Gemische in üblicher Weise durch Filtrieren, Waschen, Trocknen und Vermahlen aufarbeitet.

2. Zahnpflegemittel nach Anspruch 1 dadurch gekennzeichnet, dass es eine Fällungskieselsäure mit den folgenden physikalisch-chemischen Kenndaten:

| Trocknungsverlust (DIN 53 198) | % | 3 - 7 |
|---|---|---|
| Leitfähigkeit bei 25°C (4 %ige Aufschlämmung) | μS | 400 - 800 |
| pH-Wert ( 5 % nach DIN 53 200) | | 6 - 7 |
| BET-Oberfläche (DIN 66 131) | $m^2$/g | 130 - 150 |
| Stampfdichte (DIN 53 194) | g/l | 100 - 150 |
| Makroporenvolumen D >3 nm (nach der Hg-Einpreßmethode) | ml/g | 3,2 - 4,0 |
| ölaufnahme n. Gardner | ml/100g | 180 - 200 |
| Wasserrückhaltevermögen | % | 76 - 79 |
| Cu-Abrieb | mg | 5 - 14 |
| RDA-Abrieb | | 35 - 100 |
| REA-Abrieb | | 40 - 90 |
| Kratzer | | wenig - sehr wenige |
| Viskosität (in 16 %iger Glyzerin-Wasser-Dispersion 1:1 Spindel D mit Helipath) | mPas | 5000 - 10 000 |
| Fe-Gehalt | . ppm | 240 - 280 |

enthält.

3. Zahnpflegemittel nach Anspruch 2, <u>dadurch gekennzeichnet</u> dass man die Fällungskieselsäure mit den physikalisch-chemischen Kenndaten:

| Trocknungsverlust (DIN 53 198) | % | 3 - 7 |
|---|---|---|
| Leitfähigkeit bei 25°C (4 %ige Aufschlämmung) | μS | 400 - 800 |
| pH-Wert ( 5 % nach DIN 53 200) | | 6 - 7 |
| BET-Oberfläche (DIN 66 131) | $m^2$/g | 130 - 150 |
| Stampfdichte (DIN 53 194) | g/l. | 100 - 150 |
| Makroporenvolumen D >30 nm (nach der Hg-Einpreßmethode) | ml/g | 3,2 - 4,0 |
| ölaufnahme n. Gardner | ml/100g | 180 - 200 |
| Wasserrückhaltevermögen | % | 76 - 79 |
| Cu-Abrieb | mg | 5 - 14 |
| RDA-Abrieb | | 35 - 100 |
| REA-Abrieb | | 40 - 90 |
| Kratzer | | wenig - sehr wenige |
| Viskosität ( in 16 %iger Glyzerin-Wasser-Dispersion 1:1 Spindel D mit Helipath) | mPas | 5000 - 10 000 |
| Fe-Gehalt | ppm | 240 - 280 |

dadurch hergestellt, dass man zum einen eine Verdickungsfällungskieselsäuresuspension durch Umsetzung einer Alkalisilikatlösung mit einer Säure unter Vermeidung einer Gelbildung herstellt, wobei zur Fällung der Kieselsäure in eine vorgelegte Alkalisilikatlösung mit einer Konzentration von etwa 5 - 25 g $SiO_2$ je Liter Lösung die Säure und die Alkalisilikatlösung mit bestimmten Lösungskonzentrationen und bestimmten Zulaufgeschwindigkeiten unter Aufrechterhaltung einer Fälltemperatur im Reaktionsmedium zwischen 80 und 90° C, derarting eingespeist werden, dass die Viskosität des Reaktionsmediums in einem Zeitraum von mindestens 30 % der gesamten Fälldauer gleichmässig niedrig und der pH-Wert zwischen 10 und 12 gehalten wird, und die Zugabe der Reaktionsteilnehmer beendet wird, bevor die Viskosität nach Durchlaufen eines Maximums auf einen weniger als 100 % über der Anfangsviskosität liegenden Wert gesunken ist, und der pH-Wert mit Schwefelsäure unter 7 eingestellt wird, zum anderen eine Abrasivfällungskieselsäuresuspension herstellt, indem man eine Originalfällungskieselsäuresuspension, die durch Fällung der Kieselsäure aus einer vorgelegten Alkalisilikatlösung mit einer Konzentration von etwa 5-25 g $SiO_2$ je Liter Lösung mit einer Säure und Alkalimetallsilikatlösung mit bestimmten Lösungskonzentrationen und bestimmten Zulaufgeschwindigkeiten unter Aufrechterhaltung einer Fälltemperatur im Reaktionsmedium zwischen 80 und 90° C, wobei gegebenenfalls der Behälterinhalt während der gesamten Fälldauer intensiv geschert wird, derart hergestellt wird, dass die Viskosität des Reaktionsmediums in einem Zeitraum von mindestens 30 % der gesamten Fälldauer gleichmässig niedrig under der pH-Wert zwischen 10 und 12 gehalten wird, und die Zugabe der Reaktionsteilnehmen beendet wird, bevor die Viskosität nach Durchlaufen eines Maximums auf einen weniger als 100 % über der Anfangsviskosität liegenden Wert gesunken ist, den pH-Wert mit Schwefelsäure auf 7 bis 9 einstellt, mit heissem Wasser auf einen Fällungskieselsäuregehalt von 10 - 30 g/l und Natriumsulfatgehalt von 6 - 20g $Na_2SO/1$ verdünnt, auf 80 - 90° C erwärmt, und unter Konstanthalten dieses pH-Wertes durch gleichzeitigen Zulauf von Alkalimetallsilikatlösung, Schwefelsäure und gegebenenfalls heissem Wasser über eine Fälldauer von 15 bis 180 Minuten eine Fällungskieselsäureendkonzentration von 40 bis 80 g/l einstellt, die Suspension mit Schwefelsäure auf einen pH-Wert unter 7 ansäuert, beide Fällungskieselsäuresuspensionen miteinander vermischt, die Fällungskiesalsäure abfiltriert, wäscht, spühtrocknet und anschliessend mit der Luftstrahlmühle vermahlt.

4. Zahnpflegemittel nach den Ansprüchen 1 bis 3, dadurch gekennzeichnet, dass es neben 30-50 % Putzkörper 50-70 % eines Lösungsmittelgemisches Glyzerin zu Wasser 1:1 enthält.

5. Zahnpflegemittel nach Anspruch 4, dadurch gekennzeichnet, dass der Putzkörper mehr Fällungskieselsäure als Perlit enthält.

**Claims**

1. Dental care medium for prophylatic dental hygiene, characterised in that as polishing substance it contains a mixture consisting of a perlite and a synthetically produced precipitation salicic acid whereby the precipitation salicic acid is obtained in that the precipitation salicic acids of different particle sizes and particle density obtained in the usual manner on the precipitation path are homogeneously mixed with one another in the suspension phase and the mixtures treated in the usual manner by filtering, washing, drying and grinding.

2. Dental care medium according to claim 1, characterised in that it contains a precipitation salicic acid with the following physical-chemical characteristics:

| | | |
|---|---|---|
| Drying loss (DIN 53 198) | % | 3 - 7 |
| Conductivity at 25°C (4% suspension) | ₁ us | 400 - 800 |
| pH-value (5% according to DIN 53 200 | | 6 - 7 |
| BET surface (DIN 66131) | $m^2/g$ | 130 - 150 |
| Compressed density (DIN 53 194) | g/l | 100 - 150 |
| Macropore volume D>30nm (according to the Hg-press in method) | ml/g | 3.2 - 4.0 |
| Oil absorption acc. to Gardner | ml/100g | 180 - 200 |
| Water retention capacity | % | 76 - 79 |
| Cu - absrasion | mg | 5 - 14 |
| RDA - abrasion | | 35 - 100 |
| REA - abrasion | | 40 - 90 |
| Scratches | | few - very few |
| Viscosity (in 16% glycerin-water dispersion 1:1 spindle D with Helipath) | mPas | 5000 - 100000 |
| Fe content | ppm | 240 - 280 |

3. Dental care means according to claim 2, characterised in that the precipitation salicic acid with the physical-chemical characteristics:

| | | |
|---|---|---|
| Drying loss (DIN 53 198) | % | 3 - 7 |
| Conductivity at 25°C (4% suspension) | µs | 400 - 800 |
| pH value (5% acc. to DIN 53 200) | | 6 - 7 |
| BET surface (DIN 66131) | m$^2$/g | 130 - 150 |
| Compressed density (DIN 53194) | g/l | 100 - 150 |
| Macropore volume D >30nm (according to the Hg press in method) | ml/g | 3.2 - 4.0 |
| Oil absorption acc. to Gardner | ml/100g | 180 - 200 |
| Water retention capacity | % | 76 - 79 |
| Cu - abrasion | mg | 5 - 14 |
| RDA abrasion | | 35 - 100 |
| REA abrasion | | 40 - 90 |
| Scratches | | few - very few |
| Viscosity (in 16% glycerin-water -dispersion 1:1 Spindle D with helipath) | mpas | 5000 - 10000 |
| Fe - content | ppm | 240 - 280 |

is produced in that on the one hand a thickening precipitation salicic acid suspension is produced by transformation of an alkaline silicate solution with an acid avoiding gel formation whereby for the precipitation of the salicic acid in a prepared alkaline silicate solution with a concentration of about 5 - 25 g SiO$_2$ per litre of solution the acid and the alkaline silicate solution are fed with certain solution concentrations and certain flow velocities maintaining a precipitation temperature in the reaction medium between 80 and 90°C such that the viscosity of the reaction medium in a space of time of at least 30% of the whole precipitation time is kept uniformly low and the pH value between 10 and 12 and the addition of the reaction participants is terminated before the viscosity, after passing through a maximum, has dropped to a value less than 100% above the initial viscosity and the pH value with sulphuric acid is suspended below 7 and on the other hand an abrasive percipitation salicic acid suspension is produced in that an original precipitation salicic acid suspension which by precipitation of the salicic acid from a prepared alkaline silicate solution with a concentration of about 5.25 g SiO$_2$ per litre of solution with an acid and alkaline metal silicate solution with certain solution concentrations and certain flow velocities maintaining a precipitation temperature in the reaction medium between 80 and 90°C whereby as the case may be, the container content is intensively sheared, is produced such that the viscosity of the reaction medium in a space of time of at least 30% of the whole precipitation time is kept uniformly low under the pH value between 10 and 12 and the addition of the reaction participants is terminated before the viscosity after passing through a maximum has dropped to a value less than 100% above the initial viscosity, and the pH value with sulphuric acid is set at 7 to 9, with hot water to a precipitation salicic acid content of 10-30 g/l and sodium sulphate content of 6-20g Ha$_2$ SO/1, heated to 80 - 90°C and keeping constant this pH value by simultaneous influx of alkaline metal solution, sulphuric acid and as the case may be hot water over a precipitation time of 15 to 180 minutes sets a precipitation salicic acid concentration of 40 to 80 g/l which acidifies the suspension with sulphuric acid to a pH value below 7, mixes with one another both precipitation salicic acid suspensions, filters, washes, spray dries and then grinds with the air jet mill the precipitation salicic acid.

4. Dental care medium according to claims 1 to 3, characterised in that besides 30 - 50% polishing substance it contains 50 - 70% of a solvent mixture glycerin to water 1:1.

5. Dental care medium according to claim 4, characterised in that the polishing body contains more precipitation salicic acid than perlite.

**Revendications**

1. Produit pour l'hygiène des dents, destiné à l'hygiène dentaire caractérisé en ce qu'il contient en tant qu'agent nettoyant un mélange constitué d'une perlite et d'une silice précipitée obtenue par synthèse, la silice précipitée étant obtenue par le fait que l'on mélange dans la phase en suspension, jusqu'à homogénéité, les silices précipitées obtenues par précipitation d'une manière usuelle, présentant des granulométries et des masses volumiques de particules différentes, les mélanges étant traités d'une manière usuelle par filtration, lavage, séchage et broyage.

2. Produit pour l'hygiène des dents, selon la revendication 1, caractérisé en ce qu'il contient une silice précipitée ayant les caractéristiques physico-chimiques suivantes :

| | | |
|---|---|---|
| Perte à la dessication (DIX 53 198) | % | 3-7 |
| Conductivité à 25°C (suspension à 4 %) | $\mu s$ | 400-800 |
| pH (5 % selon DIN 53 200) | | 6-7 |
| Aire BET (DIX 66 131) | $m^2/g$ | 130-150 |
| Masse volumique après tassement (DIX 53 194) | g/l | 100-150 |
| Volume des macropores D > 30 nm (par la méthode d'introduction du mercure) | ml/g | 3,2-4,0 |
| Absorption d'huile selon Gardner | ml/100 g | 180-200 |
| Pouvoir de rétention d'eau | % | 76-79 |
| Abrasion du Cu | mg | 5-14 |
| Indice d'abrasion RDA | | 35-100 |
| Indice d'abrasion REA | | 40-90 |
| Rayures | | peu - très peu |
| Viscosité (dans une dispersion 1:1 de glycérol à 16 % et d'eau, broche D avec helipath) | mPas | 5.000-10.000 |
| Teneur en Fe | ppm | 240-280 |

3. Produit pour l'hygiène des dents selon la revendication 2, caractérisé en ce qu'on prépare la silice précipitée ayant les caractéristiques physico-chimiques suivantes :

| | | |
|---|---|---|
| Perte à la dessication (DIN 53 198) | % | 3-7 |
| conductivité à 25°C (suspension à 4 %) | $\mu s$ | 400-800 |
| pH (5 % selon DIN 53 200) | | 6-7 |
| Aire BET (DIN 66 131) | $m^2/g$ | 130-150 |
| Masse volumique après tassement (DIN 53 194) | g/l | 100-150 |
| Volume des macropores D > 30 nm (par la méthode d'introduction du mercure) | ml/g | 3,2-4,0 |
| Absorption d'huile selon Gardner | ml/100 g | 180-200 |
| Pouvoir de rétention d'eau | % | 76-79 |
| Abrasion du Cu | mg | 5-14 |
| Indice d'abrasion RDA | | 35-100 |
| Indice d'abrasion REA | | 40-90 |
| Rayures | | peu - très peu |
| Viscosité (dans une dispersion 1:1 de glycérol à 16 % et d'eau, broche D avec helipath) | mPas | 5.000-10.000 |
| Teneur en Fe | ppm | 240-280 |

par le fait que, d'une part, on prépare une solution épaississante de silice précipitée, par réaction de solutions de silicate de métaux alcalins avec un acide, en évitant la formation d'un gel, auquel cas pour la précipitation de la silice, on introduit dans une solution de silicate de métal alcalin, à une concentration d'environ 5 à 25 g de $SiO_2$ par litre de solution, l'acide et la solution de métal alcalin, avec certaines concentrations de la solution et avec certaines vitesses d'arrivée, en maintenant une température de précipitation, dans le milieu réactionnel, comprise entre 80 et 90°C, l'introduction se faisant de façon que la viscosité du milieu réactionnel soit maintenue uniformément basse pendant un laps de temps d'au moins 30 % de la durée totale de la précipitation, le pH étant maintenu entre 10 et 12 pendant ce même laps de temps, l'addition des substances participant à la réaction étant terminée avant que la viscosité, après être passée par un maximum, ne tombe à une valeur supérieure de moins de 100 % à la viscosité initiale et que le pH soit ajusté à une valeur inférieure à 7 à l'aide d'acide sulfurique ; d'autre part, on prépare une suspension abrasive de silice précipitée et pour cela, on prépare une suspension originale de silice précipitée par précipitation de la silice à partir d'une solution de silicate d'un métal alcalin à une concentration d'environ 5 à 25 g de $SiO_2$ par litre de solution, avec un acide et une solution de silicate de métal alcalin présentant des concentrations données en solution, avec certaines vitesses d'arrivée et en maintenant une température de précipitation dans le milieu réactionnel comprise entre 80 et 90°C, le contenu du réacteur étant éventuellement soumis à un cisaillement intense pendant la totalité de l'opération de précipitation, la préparation étant mise en oeuvre de telle sorte que la viscosité du milieu réactionnel soit maintenue uniformément basse pendant un laps de temps d'au moins 30 % de la durée totale de la précipitation, le pH étant maintenu entre 10 et 12 pendant ce laps de temps, l'addition des substances participant à la réaction étant terminée avant que la viscosité, après être passée par un maximum, ne tombe à une valeur de moins de 100 % supérieure à la viscosité initiale ; on ajuste le pH à 7-9 avec de l'acide sulfurique ; on dilue à l'eau chaude jusqu'à une teneur en silice précipitée de 10 à 30 g par litre et une teneur en sulfate de sodium de 6 à 20 g de $Na_2SO_4/l$ ; on chauffe à 80-90°C et en maintenant ce pH constant par addition simultanée d'une solution d'un silicate de métal alcalin, d'acide sulfurique et éventuellement d'eau chaude pendant une durée de précipitation de 15 à 180 minutes, on ajuste la concentration finale de la silice précipitée à 40-80 g/l ; on acidifie la suspension à l'acide sulfurique jusqu'à un pH inférieur à 7 ; on mélange l'une à l'autre les deux solutions de silice précipitée ; puis on sépare par filtration, on lave et on sèche par atomisation la silice précipitée, et enfin on la broie au broyeur à jet d'air.

4. Produit pour l'hygiène des dents selon les revendications 1 à 3, caractérisé en ce qu'il contient, outre 30 à 50 % d'un produit nettoyant, 50 à 70 % d'un mélange de solvants constitué d'un mélange 1:1 de glycérol et d'eau.

5. Produit pour l'hygiène des dents selon la revendication 4, caractérisé en ce que l'agent nettoyant contient plus de silice précipitée que de perlite.

Fig. 1

Fig. 2

# *Fig. 3*  AUF DEM MARKT GÄNGIGE PROPHYLAXEPASTEN

EP 0 268 763 B1

| | HART 0' | HART 30' | MITTELHART 0' | MITTELHART 30' | WEICH 0' | WEICH 30' |
|---|---|---|---|---|---|---|
| $RZ$ | 0.138 µm | 2.070 µm | 0.177 µm | 0.876 µm | 0.138 µm | 0.843 µm |
| $RA$ | 0.033 µm | 0.701 µm | 0.038 µm | 0.354 µm | 0.032 µm | 0.262 µm |
| $RS$ | 0.034 µm | 0.830 µm | 0.059 µm | 0.421 µm | 0.039 µm | 0.323 µm |
| $RP$ | 0.076 µm | 2.129 µm | 0.208 µm | 0.781 µm | 0.181 µm | 0.449 µm |
| $ZS$ | 0.023 µm | 1.110 µm | 0.080 µm | 0.335 µm | 0.057 µm | 0.243 µm |
| $Z1$ | 0.115 µm | 3.447 µm | 0.227 µm | 0.784 µm | 0.227 µm | 0.991 µm |
| $Z2$ | 0.132 µm | 1.836 µm | 0.254 µm | 0.586 µm | 0.098 µm | 1.125 µm |
| $Z3$ | 0.142 µm | 2.883 µm | 0.098 µm | 1.367 µm | 0.151 µm | 0.730 µm |
| $Z4$ | 0.176 µm | 1.538 µm | 0.275 µm | 1.055 µm | 0.083 µm | 0.872 µm |
| $Z5$ | 0.127 µm | 0.645 µm | 0.083 µm | 0.586 µm | 0.129 µm | 0.496 µm |

PROFIL R
↑ 10mm  100 µm
→ 10mm 1.000 µm

PROFIL R
↑ 10mm  100 µm
→ 10mm 1.000 µm

PROFIL R
↑ 10mm  100 µm
→ 10mm 1.000 µm

PROFIL R
↑ 10mm  100 µm
→ 10mm 1.000 µm

PROFIL R
↑ 10mm  100 µm
→ 10mm 1.000 µm

PROFIL R
↑ 10mm  100 µm
→ 10mm 1.000 µm

0'  30'  0'  30'  0'  30'

HART            MITTELHART            WEICH

EP 0 268 763 B1

## _Fig. 4_ PROPHYLAXEPASTEN MIT FÄLLUNGSKIESELSÄURE

| | HART | | | MITTELHART | | | WEICH | |
|---|---|---|---|---|---|---|---|---|
| RZ 0,091 µm | RZ 0,569 µm | RZ 0,129 µm | RZ 0,588 µm | RZ 0,081 µm | RZ 0,461 µm |
| RA 0,024 µm | RA 0,125 µm | RA 0,032 µm | RA 0,111 µm | RA 0,018 µm | RA 0,079 µm |
| RS 0,024 µm | RS 0,148 µm | RS 0,039 µm | RS 0,139 µm | RS 0,020 µm | RS 0,101 µm |
| RP 0,059 µm | RP 0,364 µm | RP 0,076 µm | RP 0,325 µm | RP 0,046 µm | RP 0,198 µm |
| ZS 0,021 µm | ZS 0,177 µm | ZS 0,045 µm | ZS 0,185 µm | ZS 0,031 µm | ZS 0,087 µm |
| Z1 0,125 µm | Z1 0,881 µm | Z1 0,125 µm | Z1 0,347 µm | Z1 0,083 µm | Z1 0,464 µm |
| Z2 0,083 µm | Z2 0,540 µm | Z2 0,120 µm | Z2 0,745 µm | Z2 0,134 µm | Z2 0,391 µm |
| Z3 0,078 µm | Z3 0,483 µm | Z3 0,208 µm | Z3 0,791 µm | Z3 0,066 µm | Z3 0,593 µm |
| Z4 0,100 µm | Z4 0,474 µm | Z4 0,100 µm | Z4 0,476 µm | Z4 0,068 µm | Z4 0,481 µm |
| Z5 0,071 µm | Z5 0,469 µm | Z5 0,095 µm | Z5 0,581 µm | Z5 0,054 µm | Z5 0,374 µm |

PROFIL R
↑10mm 100 µm
−10mm 1,000 µm

0′    30′    0′    30′    0′    30′